# EUROPÄISCHE PATENTSCHRIFT

(11) **EP 0 698 787 B1**
(45) Veröffentlichungstag und Bekanntmachung des Hinweises auf die Patenterteilung: **14.01.2004**
(21) Anmeldenummer: 95113326.3
(22) Anmeldetag: 24.08.1995
(51) Int. Cl.: G01N 27/327, C12Q 1/00, G01N 33/543, C07C 323/58, C07F 9/50, C07D 207/44

(54) **Elektrochemischer Sensor**
Electrochemical sensor
Capteur électrochimique

(30) Priorität: 24.08.1994 DE 4430023
(43) Veröffentlichungstag der Anmeldung: 28.02.1996
(73) Patentinhaber: Roche Diagnostics GmbH, 68305 Mannheim (DE)
(72) Erfinder: Rüger, Petra, Dr., D-82377 Penzberg (DE); Ambrosius, Dorothee, Dr., D-81477 München (DE); Schmidt, Bernd, Dr., D-81476 München (DE); Sluka, Peter, Dr., D-82362 Weilheim (DE); Guder, Hans-Joachim, Dr., D-67269 Grünstadt (DE); Kopetzki, Erhard, Dr., D-82377 Penzberg (DE)
(74) Vertreter: Weiss, Wolfgang, Dipl.-Chem. Dr.

(56) Entgegenhaltungen:
- EP-A- 0 524 722
- EP-A- 0 583 894
- EP-A- 0 584 794
- WO-A-89/12624
- WO-A-92/08788
- WO-A-92/10757
- US-A- 4 053 651
- CHEMICAL ABSTRACTS, vol. 84, no. 20, 17.Mai 1976 Columbus, Ohio, US; abstract no. 137608h, O. OKUMURA ET AL. 'Cleaning composition' Seite 122; & JP-A-07 602 708 10.Januar 1976
- ANALES DE QU MICA, Bd. 75, Nr. 6, 1979 Seiten 471-475, A. MEDEROS ET AL. 'El acido dicarboximetil-N,N-Metionina'

## Beschreibung

Die Erfindung betrifft einen elektrochemischen Sensor, enthaltend ein Trägermaterial, das eine Edelmetalloberfläche aufweist, und ein an die Edelmetalloberfläche adsorptiv gebundenes enzymatisch aktives Protein.

Der Einsatz von Enzymsensoren zur Bestimmung eines Analyten durch elektrochemische Messung ist bekannt (siehe z.B. Turner et al., Biosensors, Fundamentals and Application (1987), Oxford University Press).

Die Immobilisierung von Enzymen auf der Sensoroberfläche erfolgte bisher in allgemeinen durch Einschluß hinter und in Membranen, Bindung an Polymere und Quervernetzung mit bifunktionellen Reagenzien. Diese Methoden haben den Nachteil, daß sich die Enzymmoleküle statistisch verteilt in einer dicken Schicht auf der Festphase befinden. Damit ist die Diffusion des Analyten zum reaktiven Zentrum des Enzyms sowie die Diffusion des Produkts zur Elektrode gehemmt, die Reaktionszeit des Sensors ist lang und die Empfindlichkeit ist gering. Ein weiterer Nachteil dieser Art der Immobilisierung ist, daß es bei kovalenter Bindung des Enzyms an die Sensoroberfläche oft zu Aktivitätsverlusten aufgrund von Veränderungen des aktiven Zentrums kommt. Die obengenannten Methoden zur Bindung des Enzyms an die Festphase sind beispielsweise bei A. Wiseman (Handbook of Enzyme Biotechnology, Alice Horwood, Chichester (1985), 2.Aufl., 166 ff.) oder O. Zaborsky (Immobilised Enzymes, CRC Press, Cleveland Ohio (1974), 2. Aufl.) beschrieben.

Seit einiger Zeit ist die Herstellung von definierten dünnen Monoschichten auf Festphasen und die Verwendung dieser Monoschichten zur Immobilisierung von biologischen Komponenten bekannt. So beschreiben z.B. Lee et al. (Sens. Act. B, 12 (1993), 153-158) Langmuir-Blodgett-Membranen zur Adsorption von Avidin, an das anschließend eine mit Biotin markierte Glucose-Oxidase gebunden werden kann. FR-A-2682765 offenbart das gemeinsame Aufbringen von Glucose-Oxidase und amphiphilen Molekülen im Langmuir-Blodgett-Verfahren. Die Verwendung von Lipid-Doppelschichten in Sensoren ist auch bei Tien et al. (Crit. Rev. Biomed. Eng., 18 (1991) 323-340) beschrieben. Ein Nachteil dieser Langmuir-Blodgett-Schichten besteht in ihrer geringen Stabilität.

Eine weitere Möglichkeit zur Herstellung molekularer dünner Schichten ist die sogenannte SAM (selbst-assemblierende Monoschicht)-Technik, die von Bain und Whitesides (Ang. Chem. 101 (1988), 522-528) beschrieben wurde. Solche selbst-assemblierenden Monoschichten entstehen beispielsweise durch Adsorption langkettiger Thiole auf Goldoberflächen. Von Sawaguchi et al. (Bioelectrochemistry and Bioenergetics 29 (1992), 127-133) und Willner et al. (J. Am. Chem.Soc. 114 (1992), 10965-10966) wurde die Immobilisierung von Diaphorase und Gluthationreduktase an eine Metalloberfläche über Thiole beschrieben. Collinson und Bowden (Langmuir 8 (1992), 1247-1250) offenbaren die Immobilisierung von Cytochrom C an eine Metalloberfläche unter Verwendung von 16-Mercaptohexadecansäure und 11-Mercaptoundecansäure. Es wurde gefunden, daß nur ein Drittel der Oberfläche mit Cytochrom C belegt ist. Kinnear und Monbouquette (Langmuir 9 (1993), 2255-2257) beschreiben den Einbau eines direkt auf der Metalloberfläche absorbierten Enzyms in eine selbst-assemblierende Monoschicht.

Die bisher beschriebenen Methoden zur Immobilisierung von Enzymen in selbst-assemblierenden Monoschichten haben den Nachteil einer geringen Belegungsdichte des Enzyms und einer geringen Leitfähigkeit und damit verbunden einer geringen Sensitivität der resultierenden Monoschicht. Insbesondere sind die Probleme dadurch bedingt, daß bei Verwendung langkettiger Thiole der Abstand von Enzym zur Metalloberfläche zu groß ist und bei Verwendung kurzkettigerer Thiole die Homogenität der Oberfläche stark abnimmt. Auch die direkte Adsorption des Enzyms auf der Metalloberfläche führt nicht zu einer ausreichend homogenen Schicht und überdies kann bei der direkten Adsorption auf dem Metall eine teilweise Denaturierung der Proteine erfolgen.

Aus WO 92/10757 ist eine Bindematrix bekannt, enthaltend ein Trägermaterial und einen daran über Ankergruppen adsorbierten Festphasenreaktanten, der mit mindestens einem freien Reaktionspartner bindefähig ist, dadurch gekennzeichnet, daß der Festphasenreaktant eine verdünnte und im wesentlichen lateral homogene Bindeschicht auf der Oberfläche des Trägermaterials bildet. Die in WO 92/10757 beschriebenen Festphasen-Reaktanten sind beispielsweise Biotin oder Haptene. Es sind keine Beispiele beschrieben, in denen Enzyme als Festphasenreaktanten verwendet werden.
In EP-A-0 584 794 wird ein elektrochemischer Sensor beschrieben, bei dem ein Redoxenzym über ein Bindemolekül auf einer Edelmetalloberfläche immobilisiert ist.
Das der vorliegenden Erfindung zugrundeliegende Problem bestand darin, einen elektrochemischen Sensor bereitzustellen, bei dem die Nachteile des Standes der Technik zumindest weitgehend beseitigt sind. Insbesondere sollte der Sensor eine hohe Belegungsdichte des Enzyms verbunden mit einer hohen Leitfähigkeit und Sensitivität liefern.

Das erfindungsgemäße Problem wird gelöst durch einen elektrochemischen Sensor, enthaltend ein Trägermaterial, das eine Edelmetalloberfläche aufweist, eine adsorptiv an das Trägermaterial gebundene und im wesentlichen lateral homogene Monoschicht auf der Oberfläche des Edelmetalls , wobei die Monoschicht Bindemoleküle umfaßt, die über Ankergruppen an das Edelmetall gebunden sind, wobei die Bindemoleküle über eine ionische Bindung, eine kovalente Bindung oder eine Metallchelatbindung mit einem enzymatisch aktiven Protein verknüpft sind und wobei der Belegungsgrad der Bindemoleküle auf dem Trägermaterial geringer als der maximale Belegungsgrad ist und wobei das enzymatisch aktive Protein in einer dichtgepackten Schicht liegt. Der Belegungsgrad der Bindemoleküle ist geringer als der maximale Belegungsgrad, d.h. es liegt eine bezüglich der Bindemoleküle verdünnte Monoschicht vor. Über den Belegungsgrad der Bindemoleküle kann die Belegung der Festphase mit dem enzymatisch aktiven Protein optimiert werden. Im Optimum ist eine dichtgepackte Enzymschicht realisiert. Der Belegungsgrad der Monoschichten mit den Bindemolekülen ist kleiner als 100% und günstigerweise 1-99%, bevorzugt 2-98% und besonders bevorzugt 5-95%.

Der erfindungsgemäße elektrochemische Sensor enthält aufgrund der auf dem Trägermaterial adsorbierten verdünnten Schicht an Bindemolekülen eine monomolekulare Schicht von vorzugsweise einheitlich ausgerichteten enzymatisch aktiven Proteinen bzw. Enzymen mit maximaler Belegungsdichte auf der Festphase, wodurch die Ansprechzeit des Sensors verringert und die Signalausbeute pro Fläche erhöht wird. Ein weiterer Vorteil des Sensors besteht darin, daß die Monoschicht durch ein einfaches Tauchverfahren aufgebracht werden kann. Außerdem eignet sich der erfindungsgemäße Sensor aufgrund der kovalenten Bindung der Enzyme auch sehr gut zur Mehrfach- und Dauermessung.

Das Trägermaterial des erfindungsgemäßen Sensors weist eine Edelmetalloberfläche auf, wobei grundsätzlich alle Edelmetalle oder Edemetallgemische bzw. Edelmetall-Legierungen mit einem Standard-Elektrodenpotential (d.h. einem Potential gegenüber einer Normalwasserstoffelektrode Pt/H₂ (1 atm)/H⁺ (a = 1)) als Bezugselektrode, das positiver als das Potential einer Silberelektrode ist. Beispiele für geeignete Edelmetalle sind Au, Pt, Pd, etc.. Besonders bevorzugt weist das Trägermaterial eine Gold- oder Palladiumoberfläche auf.

Die Herstellung eines Trägermaterials mit einer Edelmetalloberfläche geschieht beispielsweise durch Bedampfen von Glas mit Chrom als Haftvermittler, wobei eine ca. 0,1 - 10 nm dicke Schicht entsteht. Diese Chromschicht wird anschließend mit dem Edelmetall, z.B. Gold, bedampft, wobei eine Schicht entsteht, welche die Oberfläche des Trägermaterials darstellt. Diese Edelmetallschicht hat vorzugsweise eine Dicke zwischen 10-200 nm. Bei einem Trägermaterial aus Kunststoff (z.B. Polycarbonat) ist auch ein direktes Aufdampfen des Edelmetalls auf den Träger möglich.

Die Adsorption der Moleküle an die Oberfläche des Trägermaterials wird durch Ankergruppen vermittelt. Die Art der Ankergruppen hängt von der jeweiligen Oberfläche des Trägermaterials ab. Als Ankergruppen sind insbesondere Thiol-, Disulfid-, oder/und Phosphingruppen geeignet. Thiol- oder/und Disulfidgruppen sind beispielsweise als Ankergruppen für eine Goldoberfläche und Phosphingruppen als Ankergruppen für eine Palladiumoberfläche besonders geeignet.

Für unterschiedliche Methoden zur Herstellung verdünnter Bindeschichten wird auf WO 92/10757 verwiesen. In einer ersten Ausführungsform besteht die Monoschicht aus einer einzigen Molekülsorte, wobei die Oberfläche mit Ankergruppen nicht vollständig belegt ist. Die Herstellung einer derartigen Schicht kann beispielsweise durch Eintauchen der Oberfläche in eine Lösung erfolgen, die eine sehr geringe Konzentration (z.B < 1 x 10⁻⁵ mol/l) an Bindemolekülen enthält. Auf diese Weise wird ein verdünnter Film von Bindemolekülen auf der Oberfläche erhalten, auf dem anschließend das Enzym immobilisiert werden kann.

In einer bevorzugten Ausführungsform der Erfindung umfaßt die Monoschicht neben den Bindemolekülen noch weiterhin Verdünnungsmoleküle, die über Ankergruppen adsorptiv an das Edelmetall gebunden und nicht mit einem enzymatisch aktiven Protein verknüpft sind. Die Herstellung einer monomolekularen Schicht aus Binde- und Verdünnungsmolekülen erfolgt durch Eintauchen der Oberfläche in eine Lösung, die Binde- und Verdünnungsmoleküle in gewünschten molaren Anteilen enthält. Auf dieser beschichteten Oberfläche kann anschließend die Immobilisierung des Enzyms erfolgen.

Vorzugsweise ist bei der Herstellung des Sensors das molare Verhältnis von Bindemolekülen zu Verdünnungsmolekülen im Bereich von 1:100 bis 100:1, besonders bevorzugt von 1:50 bis 50:1 und am meisten bevorzugt von 1:20 bis 20:1. In bestimmten Anwendungen des erfindungsgemäßen Sensors kann es bevorzugt sein, daß das molare Verhältnis von Bindemolekülen zu Verdünnungsmolekülen größer als 1:1 und insbesondere 5:1 bis 20:1 ist.

Durch den Einsatz von Verdünnungsmolekülen wird nicht nur eine Optimierung der Enzymbelegung erreicht, sondern auch die Leitfähigkeit der Schicht wird überraschenderweise wesentlich erhöht, was für elektrische Anwendungen ein wesentliches Kriterium ist. Weiterhin führen die Verdünnungsmoleküle zu einer verringerten unspezifischen Bindung von Komponenten aus einer mit dem Sensor in Kontakt stehenden Probelösung.

Das enzymatisch aktive Protein, das über eine ionische Bindung, eine kovalente Bindung oder eine Metallchelatbindung mit dem Bindemolekül verknüpft ist, wird vorzugsweise aus der Gruppe der Enzyme ausgewählt, die eine Reaktion katalysieren, bei der Verbindungen entstehen oder/und verbraucht werden, die elektrochemisch detektierbar sind. Eine derartige Reaktion kann elektrochemisch, z.B. potentiometrisch oder amperometrisch, verfolgt werden. Vorzugsweise sind dies Enzyme, die Reaktionen katalysieren, bei denen Ionen entstehen oder/und verbraucht werden, bei denen H₂O₂ entsteht oder/und bei denen elektrochemische Mediatoren umgesetzt werden. Beispiele für derartige Enzyme sind Oxidoreduktasen, d.h. Enzyme, die ein Substratmolekül oxidieren oder/und reduzieren können. Es sind aber auch Hydrolasen und solche Enzyme geeignet, bei deren Reaktion eine pH-Änderung erfolgt.

Ein bevorzugtes Beispiel für ein geeignetes Enzym ist eine Glucose-Oxidase (EC 1.1.3.4). Glucose-Oxidase aus dem Mikroorganismus Aspergillus niger ist beispielsweise kommerziell von der Firma Boehringer Mannheim GmbH, Mannheim, BRD, erhältlich.

Das enzymatisch aktive Protein kann jedoch auch ein rekombinantes Enzym sein, d.h. ein Enzym, das aus einer heterologen Wirtszelle gewonnen wird. Der Vorteil derartiger rekombinanter Enzyme ist, daß sie durch genetische Manipulation in modifizierter Form eingesetzt werden können, wodurch eine bessere Immobilisierung an der Sensoroberfläche möglich wird. Beispiele für geeignete Modifikationen sind N- oder/und C-terminale Anfügungen von einem oder mehreren Aminosäureresten, ausgewählt aus basischen Aminosäuren, wie etwa Arginin oder Lysin, sauren Aminosäuren wie etwa Aspartat oder Glutamat, chelatbildenden Aminosäuren, wie etwa Histidin, oder Cystein.

Das Enzym kann kovalent, ionisch oder über eine Metallchelatbindung mit dem Bindemolekül verknüpft sein. Eine ionische Bindung des Enzyms kann auf der Chemie von Anionen- bzw. Kationen-Austauschern basieren. In dieser Ausführungsform der Erfindung sind die Bindemoleküle über eine ionische Bindung mit dem enzymatisch aktiven Protein verknüpft, wobei die Bindung zwischen einer geladenen Endgruppe des Bindemoleküls und einem entgegengesetzt geladenen Bereich des enzymatisch aktiven Proteins erfolgt. So kann die geladene Endgruppe des Bindemoleküls beispielsweise mindestens eine Aminofunktion umfassen und der entgegengesetzt geladene Bereich des enzymatisch aktiven Proteins mindestens einen sauren Aminosäurerest, vorzugsweise eine Serie von z.B. 2 bis 8 Aspartat- oder Glutamatresten umfassen. Andererseits kann die geladene Endgruppe des Bindemoleküls mindestens eine Carboxylat- oder/und vorzugsweise Sulfönatfunktion umfassen und der entgegengesetzt geladene Bereich des Enzyms kann mindestens einen basischen Aminosäurerest, vorzugsweise eine Serie von z.B. 2 bis 8 Arginin- oder/und Lysinresten umfassen. Ein Vorteil der Immobilisierung von Enzymen auf diese Weise besteht darin, daß eine gerichtete Bindung des Enzyms an die Festphase ermöglicht wird. Ein weiterer Vorteil ist, daß das aktive Zentrum des Enzyms nicht beeinträchtigt wird, wenn der mit dem Bindemolekül reagierende Bereich ein am N- oder/und C-Terminus des Enzyms angefügter Abschnitt ist.

In einer weiteren Ausführungsform der Erfindung kann das Enzym kovalent mit dem Bindemolekül verknüpft sein, wobei die Bindung zwischen einer reaktiven Endgruppe des Bindemoleküls und einer reaktiven Gruppe des enzymatisch aktiven Proteins erfolgt. Ein bevorzugtes Beispiel für eine reaktive Endgruppe des Bindemoleküls ist eine Maleimidgruppe, die mit einem Cysteinrest des Enzyms reagieren kann. Ein weiteres Beispiel für eine kovalente Bindung ist die Bindung von Phenolresten des Enzyms an eine Diazonium-Gruppe im Bindemolekül. Auch die Kopplung von oxidierten Zuckerresten des Enzyms an Amino-Endgruppen der Bindemoleküle ist möglich. Eine ausführliche Übersicht über mögliche kovalente Immobilisierungsmittel, die bei den erfindungsgemäßen Sensoren eingesetzt werden könne, findet sich in den bereits genannten Literaturstellen Wiseman und Zaborsky. Auf diese Offenbarung wird hiermit Bezug genommen.

Auch die Bindung über Metallchelate ist zur Immobilisierung von Enzymen möglich, wobei die Bindung zwischen einer chelatbildenden Endgruppe des Bindemoleküls, einem Metallion und einem chelatbildenden Bereich des enzymatisch aktiven Proteins erfolgt.

Die chelatbildende Endgruppe des Bindemoleküls kann beispielsweise eine Di- oder Tricarboxylatgruppe umfassen und der chelatbildende Bereich des enzymatisch aktiven Proteins kann eine Serie von benachbarten Histidinresten umfassen. Beispiele für geeignete chelatbildende Metallionen sind z.B. Nickel-, Eisen-, Kobalt-, Kupfer- und Zinkionen. Auch bei der Bindung über Metall-Chelate ist eine gerichtete Bindung von Enzymen an die Festphase möglich.

Für einen erfindungsgemäßen Sensor ist weiterhin bevorzugt, daß das Bindemolekül zwischen der Ankergruppe und der zur Bindung mit dem enzymatisch aktiven proteinfähigen Endgruppe einen Spacer enthält. Die Länge des Spacers zwischen der Ankergruppe und der Bindestelle zum Enzym kann auf die Funktion des Sensors erheblichen Einfluß ausüben. Eine Variation der Länge kann die Leitfähigkeit sowie die Zugänglichkeit der Bindestellen für das Enzym beeinflussen. Vorzugsweise ist der Spacer eine gegebenenfalls Heteroatome, z.B. S-, N- oder/und 0-Atome, enthaltende Alkylenkette mit einer Längen von 4-30 Atomen. Besonders bevorzugt enthält der Spacer 1-4 C₂-C₄-Alkylenoxideinheiten, vorzugsweise 1-4 Ethylenoxideinheiten.

Die Verdünnungsmoleküle enthalten ebenso wie die Bindemoleküle eine zur Adsorption an die Festphase geeignete Ankergruppe, d.h. eine Thiol-, Disulfid-, oder/und Phosphingruppe. Die andere Endgruppe der Verdünnungsmoleküle ist vorzugsweise eine hydrophile Funktion, z.B. eine Hydroxyl-, eine C₁-C₄-Alkoxy, eine Amino-, eine Carboxyl-, eine Carbonsäureester- oder eine Carbonsäureamidfunktion. Zwischen der Endgruppe und der Ankergruppe der Verdünnungsmoleküle befindet sich vorzugsweise ebenfalls ein Spacer wie oben definiert. Besonders bevorzugt enthält der Spacer für die Verdünnungsmoleküle bis zu 15 Atome und mindestens eine Ethylenoxideinheit.

Die Herstellung von genetisch modifizierten Proteinen, die aufgrund einer Modifizierung insbesondere am C- oder/und N-Terminus einheitlich ausgerichtet an eine Festphase gebunden werden können, sowie eine Festphase mit dem ausgerichtet immobilisierten Protein wird in WO 92/08788 offenbart. Eine Metallchelat-Bindung oder eine ionische Bindung als mögliche Immobilisierungsmethode von Enzymen wird nicht offenbart. Stayton et al. (J. Am. Chem. Soc. 114 (1992), 9298-9299) beschreiben die gerichtete Bindung von Cytochrom C durch genetische Modifikation und direkte Bindung des an die Oberfläche des Enzyms eingeführten Cysteinrests an eine Goldoberfläche. Ein Nachteil dieses Verfahrens besteht darin, daß keine andere Gruppe an der Oberfläche des Enzyms vorhanden sein darf, die eine Adsorption auf der Metalloberfläche zeigt. Da aber hydrophobe Oberflächen wie Metalle oft eine starke unspezifische Adsorption von Proteinen zeigen, dürfte die Anwendung der obengenannten Enzymsensoren große Nachteile aufweisen.

Weiterhin betrifft die vorliegende Erfindung Verfahren zur Bestimmung eines Analyten in einer Probelösung, wobei man die Probelösung mit einem erfindungsgemäßen Sensor inkubiert, unter Bedingungen, die zu einer elektrochemisch nachweisbaren Reaktion des Analyten führen, und die Reaktion des Analyten durch eine elektrochemische Messung, z.B. durch eine potentiometrische oder amperometrische Messung bestimmt.

Ein bevorzugtes Beispiel für einen Analyten ist Glucose, wobei in diesem Fall ein Sensor mit einer immobilisierten Glucose-Oxidase zur Bestimmung verwendet wird. Besonders bevorzugt wird eine durch rekombinante DNA-Technologie modifizierte Glucose-Oxidase mit einer N- oder/und C-terminalen Anfügung von sauren, basischen, chelatbildenden Aminosäureresten oder/und Cysteinresten verwendet.

Noch ein weiterer Gegenstand der vorliegenden Erfindung ist eine Verbindung der allgemeinen Formel

A- (Sp-X)ₙ (I),

worin A eine HS- oder H₂P-Gruppe und n 1 bedeutet oder A eine - SS-Gruppe und n 2 bedeutet,
Sp einen Spacer mit einer Kettenlänge von 4-30 Atomen bedeutet und X eine Maleimidgruppe oder
eine Gruppe der Formel (II) 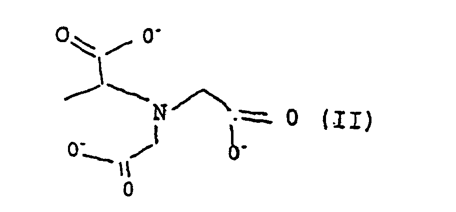 ist.

Derartige Verbindungen werden vorzugsweise zur Herstellung von elektrochemischen Sensoren verwendet.

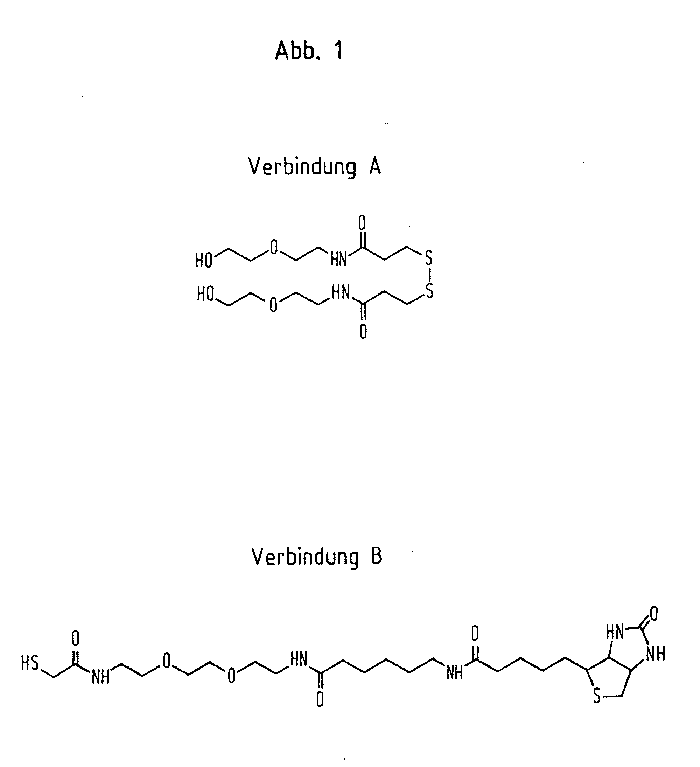

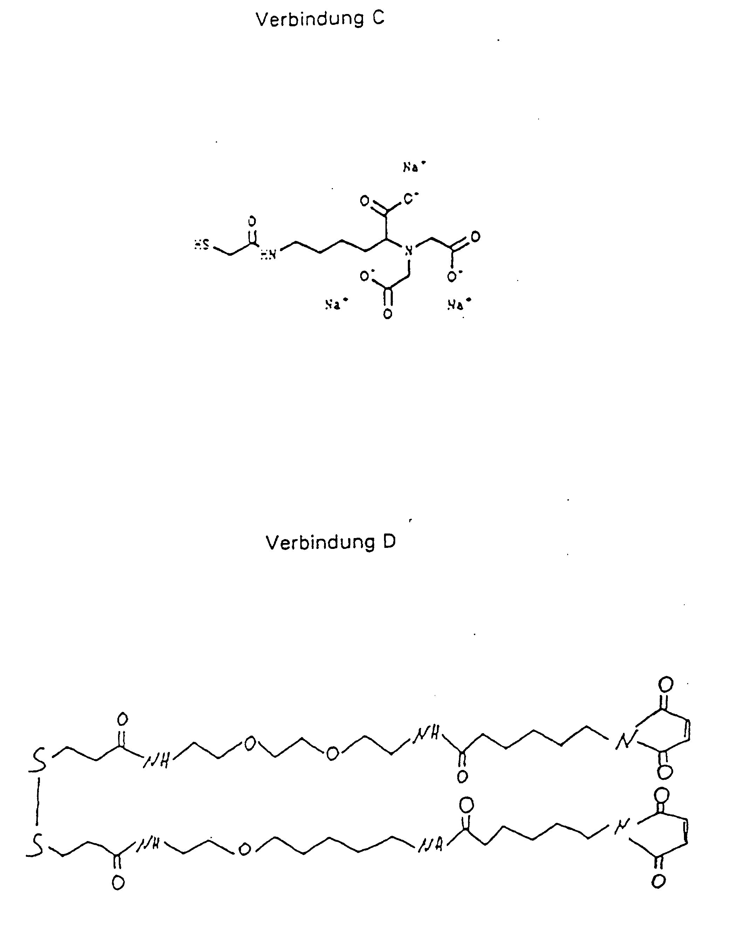

Weiterhin wird die vorliegende Erfindung durch die folgenden Beispiele in Verbindung mit den Abbildungen 1-7 und den Sequenzprotokollen erläutert.

Es zeigen:
- Abb. 1:: a) ein Verdünnermolekül (Verbindung A)
b) ein Bindemolekül mit Biotingruppe (Verbindung B)
c) ein Bindemolekül mit einer chelatbildenden Endgruppe (Verbindung C)
d) ein Bindemolekül mit einer reaktiven Maleimid-Endgruppe (Verbindung D),
- Abb. 2:: ein Cyclovoltammogramm der Sensoren
a) beschichtet mit reiner Verbindung B
b) beschichtet mit einem Gemisch aus den Verbindungen A und B (10:1),

- Abb. 3:: ein Cyclovoltammogramm der Sensoren
a) beschichtet mit reiner Verbindung C
b) beschichtet mit einem Gemisch aus den Verbindungen A und C (1:10),
- Abb. 4:: die Ergebnisse einer amperometrischen Bestimmung bei Bindung von Glucose-Oxidase-His₄ an unbeschichtete Goldoberflächen, an mit Verdünnungsmolekülen (Verbindung A) beschichtetes Gold und an mit einem chelatbildenden Bindemolekül (Verbindung C) beschichtetes Gold,
- Abb. 5:: die Abhängigkeit des Stromsignals von der Glucosekonzentration bei einem Sensor gemäß Beispiel 6,
- Abb. 6:: die Reproduzierbarkeit aufeinanderfolgender Messungen bei einem Sensor gemäß Beispiel 6,
- Abb. 7:: ein Cyclovoltammogramm der Sensoren
a) beschichtet mit reiner Verbindung D
b) beschichtet mit einem Gemisch aus den Verbindungen A und D (1:10),
- Abb. 8:: die Abhängigkeit des Stromsignals von der Glucosekonzentration bei einem Sensor gemäß Beispiel 7.

- SEQ ID NO. 1:: Den zur Konstruktion des Plasmids YEpl/AD-GOD verwendeten Primer (1) (Beispiel 1.1)
- SEQ ID NO. 2:: Den zur Konstruktion des Plasmids YEpl/AD-GOD verwendeten Primer (2) (Beispiel 1.1)
- SEQ ID NO. 3:: Den zur Konstruktion des Plasmids YEpl/GOD-(His)₄Cys verwendeten Primer (3) (Beispiel 1.3)
- SEQ ID NO. 4:: Den zur Konstruktion des Plasmids YEpl/GOD-(His)₄Cys verwendeten Primer (4) (Beispiel 1.3)
- SEQ ID NO. 5:: Den zur Konstruktion des Plasmids YEpl/GOD-Cys verwendeten Primer (5) (Beispiel 1.4)
- SEQ ID NO. 6:: Den zur Konstruktion des Plasmids YEpl/GOD-Cys verwendeten Primer (6) (Beispiel 1.4)

### Beispiel 1:

### Herstellung von modifizierten Glucose-Oxidase-(GOD)-Molekülen

### 1.1 Konstruktion des Plasmids YEpL/AD-GOD

Das Expressionsplasmid YEpL/AD-GOD leitet sich von dem Plasmid YEpL/GOD (Herstellung und Beschreibung, siehe DE-A-43 01 904, Beispiel 1.1-1.5) ab. Das Plasmid YEpL/GOD enthält das GOD-Gen aus Aspergillus niger und eignet sich zur rekombinanten Expression und Sekretion von GOD in Saccharomyces cerivisiae.

Aus konstruktionstechnischen Gründen wurde das nicht essentielle positive Regulatorgen MAL2-8cp in YEpL/GOD (stimuliert den für die GOD-Expression verwendeten α-Glucosidase Promotor) durch einen Polylinker ersetzt. Dies ermöglicht die Konstruktion von C-terminalen GOD-Fusionsproteinen mittels einer nun singulär vorhandenen Sphl Restriktionsschnittstelle.

Dazu wurde das Plasmid YEpL/GOD mit den Restriktionsendonukleasen BamHl und Mlul verdaut, das ca. 10,2 kBp lange BamHl/Mlul-YEpL/GOD-Vektorfragment isoliert und mit dem folgenden, aus 2 Oligonukleotiden durch Hybridisierung hergestellten, DNA-Linker ligiert.

Das gewünschte Plasmid YEpL/AD-GOD wurde durch Restriktionskartierung identifiziert und weiter analysiert.

### 1.2 Herstellung von GOD-(HIS)₄

Das Expressionsplasmid YEpL/GOD-(His)₄ enthält ein GOD-Gen mit 4 zusätzlichen C-terminalen His-Resten. Die Isolierung und enzymatische Charakterisierung von GOD-(His)₄ sind in DE-A-43 01 904 (Beispiele 4, 7 und 8) beschrieben.

### 1.3 Herstellung von GOD-(HIS)₄Cys

### 1.3.1. Konstruktion des Plasmids YEpL/GOD-(His)₄Cys

Das Plasmid enthält ein modifiziertes GOD Gen, das für eine GOD Enzymvariante kodiert, die C-terminal zusätzlich 4 Histidinreste und einen Cysteinrest besitzt.

Das Plasmid YEpL/GOD-(His)₄Cys wurde entsprechend wie das Plasmid GOD-(His)₄ mittels 2 komplementärer Oligonukleotide aus dem Plasmid YEpL/AD-GOD hergestellt.

Dazu wurde das Plasmid YEpL/AD-GOD mit SphI und PvuII gespalten, das ca. 10,2 kBp lange SphI/PvuII-YEpL/GOD-Vektorfragment isoliert und mit dem folgenden, aus 2 Oligonukleotiden durch Hybridisierung hergestellten, DNA Linker ligiert.

Das gewünschte Plasmid YEpL/GOD-(His)₄Cys wurde durch Restriktionskartierung (neue EcoRI Schnittstelle) identifiziert und durch partielle Sequenzierung (C-terminale Bereich des GOD Strukturgens) weiter analysiert.

### 1.3.2. Expression, Reinigung und enzymatische Charakterisierung von GOD- (His) 4Cys

Die Expression, Reinigung und enzymatische Charakterisierung erfolgte entsprechend wie für GOD-(His)₄ in DE-A-43 01 904, (Beispiele 4, 7 und 8) beschrieben.

### 1.4. Herstellung von GOD-Cys

### 1.4.1 Konstruktion des Plasmids YEpL/GOD-Cys

Das Plasmid enthält ein modifiziertes GOD Gen, das für eine GOD Enzymvariante kodiert, die C-terminal zusätzlich einen Cysteinrest besitzt.

Das Plasmid YEpL/GOD-Cys wurde entsprechend wie das Plasmid GOD-(His)₄Cys mittels 2 komplementärer Oligonukleotide aus dem Plasmid YEpL/AD-GOD hergestellt.

Dazu wurde das Plasmid YEpL/AD-GOD mit SphI und PvuII gespalten, das ca. 10,2 kBp lange SphI/PvuII-Fragment isoliert und mit dem folgenden, aus 2 Oligonukleotiden durch Hybridisierung hergestellten, DNA Linker ligiert.

Das gewünschte Plasmid YEpL/GOD-Cys wurde durch Restriktionskartierung identifiziert (neue EcoRI Schnittstelle) und durch partielle Sequenzierung (C-terminaler Bereich des GOD Strukturgens) weiter analysiert.

### 1.4.2. Expression, Reinigung und enzymatische Charakterisierung von GOD- (His)₄Cys

Die Expression, Reinigung und enzymatische Charakterisierung erfolgte entsprechend wie für GOD-(His)₄ in der DE-A-43 01 904, (Beispiele 4, 7 und 8) beschrieben.

Die C-terminalen GOD-Fusionsproteine GOD-(His)₄, GOD-(His)₄-Cys und GOD-Cys verhalten sich in Hefe (in Wildtyp und glycosylierungsdefekten Hefestämmen) hinsichtlich Sekretion, Glycosylierung, Kohlenhydratanteil, spezifischer Aktivität, Thermo- und pH-Stabilität wie das nicht modifizierte GOD Enzym.

### Beispiel 2:

### Herstellung eines Verdünnungsmoleküls

A. 2(S-Acetyl)mercaptopropionsäure-2-(2-hydroxyethoxy)ethylamid Eine Lösung von 5 g (20 mmol) N-Succinimidyl-S-acetylthiopropionat (Herstellung vgl. Beispiel 28 von W0-A-92/10757) in 50 ml THF wurden tropfenweise zu einer Lösung von 2,14 g (20 mmol) 2-(2-Aminoethoxy)ethanol in 25 ml THF im Zeitraum von 15 Min. zugegeben und für 2 Stunden bei 20°C gerührt. Nach Beendigung der Reaktion (DC-Kontrolle) wurde das Reaktionsgemisch im Vakuum eingedampft und durch Chromatographie an Silicagel gereinigt.
Ausbeute 2,7 g
DC: Kieselgel 60
Eluens: Ethylacetat/Methanol = 7:3 + 1% Essigsäure
RF = 0,67
MS (Pos FAB) : MH⁺ = 236

### B. 2-Mercaptopropionsäure-[2-(2-hydroxyethoxy)]ethylamid

600 ml einer 1 mol/l Lösung von Hydroxylamin in Methanol wurden zu 2,7 g (8,7 mmol) der unter Punkt A hergestellten Verbindung gegeben und für eine Stunde bei 20°C gerührt. Dann wurde das Lösungsmittel im Vakuum eingedampft und der Rückstand wurde dreimal mit Dichlormethan extrahiert. Es wurden 1,5 g eines öligen Rohprodukts erhalten und durch Flash-Chromatographie an Kieselgel gereinigt. Diese Verbindung ist als Verdünnungsmolekül einsetzbar.
Ausbeute: 0,86 g (farbloses Öl)
DC: Kieselgel 60,
Eluens: Dichlormethan/Methanol = 9/1
RF = 0,45
MS (pos . FAB) : MH⁺ = 194

### C. Aus der unter Punkt B hergestellten Thiolverbindung wurde durch Oxidation die Disulfidverbindung (Verbindung A) hergestellt.

### Beispiel 3

### Herstellung eines chelatbildenden Bindemoleküls (Verbindung C)

28 g Bromessigsäure wurden in 75 ml 2 N NaOH gelöst und auf 0°C abgekühlt. N-Boc-L-Lysin wurde in 75 ml 2 N NaOH gelöst und langsam zur Bromessigsäure getropft. Nach dem Zutropfen wurde das Eisbad entfernt, auf 70°C erwärmt und weitere 2 Stunden gerührt. Die Lösung wurde auf das halbe Volumen eingeengt und anschließend mit 300 ml 1 N HCl versetzt. Dabei bildete sich ein weißer Niederschlag. Der Ansatz wurde 3-4 Stunden bei 4°C stehengelassen, abgesaugt und der Rückstand 1 x mit Eiswasser gewaschen und getrocknet. Die Substanz (15,3 g) wurde anschließend in 100 ml Chloroform suspendiert. 20 ml Trifluoressigsäure wurden zugegeben und 2 Stunden bei Raumtemperatur gerührt. Chloroform und Trifluoressigsäure wurden am Hochvakuum abgezogen und der zähe Rückstand wurde mit 100 ml Ether versetzt und gerührt, bis sich ein weißer Feststoff abschied. Die Substanz wurde abgesaugt, gewaschen und getrocknet (Ausbeute 15,3 g). 1,5 g dieser Substanz wurden in 30 ml Methanol und 1,62 g Triethylamin gelöst. Unter Rühren wurden 0,92 g N-Succinimidyl-S-acetylthioacetat zugegeben. 3 Stunden wurde bei Raumtemperatur gerührt und anschließend das Lösungsmittel abgezogen. Das Produkt wurde über eine Kieselgelsäule gereinigt (Ausbeute 2 g). Dieses Produkt wurde anschließend unter Stickstoff in 40 ml 12,5%iger Ammoniaklösung gelöst und 1 Stunde bei Raumtemperatur gerührt. Die Lösung wurde am Hochvakuum eingeengt und das Produkt anschließend säulenchromatographisch isoliert (Ausbeute 500 mg).

### Beispiel 4

### Herstellung eines Bindemoleküls mit einer reaktiven Maleimidgruppe (Verbindung D)

10,9 g Dithiodipropionsäure, 12,7 g N-Hydroxysuccinimid und 22,7 g Dicyclohexylcarbodiimid wurden in 100 ml DMF und 100 ml Dioxan gelöst und 12 Stunden bei Raumtemperatur gerührt. Anschließend wurde der Niederschlag abgesaugt und das Lösungsmittel abgezogen. Man erhielt 18,5 g eines öligen Rückstandes. 2,06 g des Produkts wurden in 100 ml Dioxan gelöst. Zu dieser Lösung wurden 2,78 g Mono-N-Boc-1,8-diamino-3,6-dioxaoctan gegeben und bei Raumtemperatur 12 Stunden gerührt. Anschließend wurde der Ansatz mit 2,2 ml Trifluoressigsäure versetzt, am Rotationsverdampfer bis auf ca. 20 ml eingeengt und säulenchromatographisch gereinigt. Man erhielt nach Abziehen des Lösungsmittels ca. 4 g eines öligen Produkts. Dieses Produkt wurde in 50 ml Dioxan gelöst und zu dieser Lösung wurden 3,1 g Maleimidohexanoyl-N-hydroxy-succinimidester in 10 ml Dioxan gegeben. Der Ansatz wurde 12 Stunden bei Raumtemperatur gerührt, anschließend das Lösungsmittel abgezogen und säulenchromatographisch gereinigt. Vom Produkt wurden 190 mg als weißes Pulver erhalten.

### Beispiel 5 (Vergleich)

Auf Polycarbonatträger wurden nacheinander 2 x 40 nm Gold aufgedampft. Die Träger wurden anschließend in 5 · 10⁻⁴ mol/l eines Gemisches aus der Biotinverbindung B (Synthese: siehe Beispiel 29 von W0-A-92/10757) und Verbindung A (Synthese: siehe Beispiel 2) im molaren Verhältnis von 1:10 in Wasser 1 h lang inkubiert. Nach gründlichem Waschen in Wasser wurde im Argonstrom getrocknet.

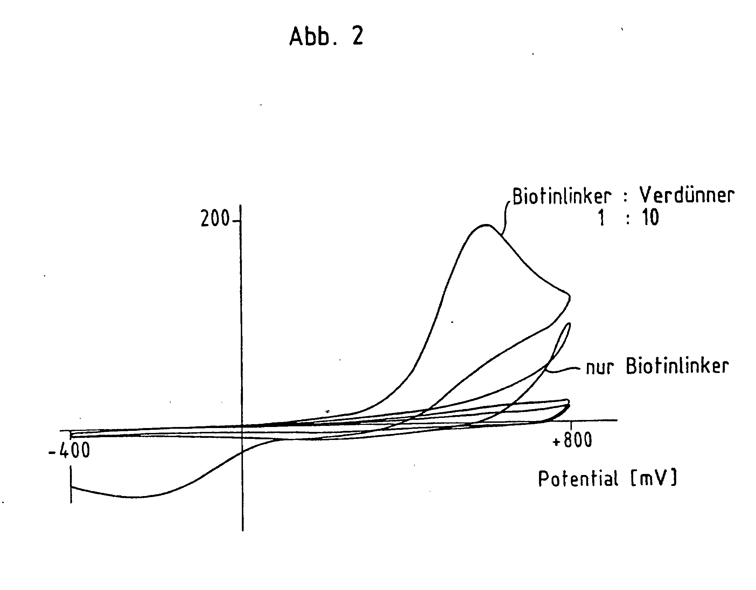

Die elektrochemische Umsetzung von 1 mmol/l K₄[Fe(CN)₆] und N,N-Dimethylnitrosoanilin wurde mit Hilfe der Cyclovoltammetrie charakterisiert. Während eine unverdünnte SAM aus der reinen Verbindung B die Umsetzung von Hexacyanoferrat nahezu vollständig verhinderte, wurde durch das Aufbringen einer Mischschicht eine gute Leitfähigkeit der Oberfläche hergestellt (Abb. 2). Oberflächenplasmonen-Resonanz- und Kontaktwinkelmessungen wiesen auf eine dichte Belegung der Oberfläche hin.

Die mit Bindemolekül (Verbindung B) und Verdünner (Verbindung A) beschichtete Elektrode wurde anschließend 1 h lang in eine Lösung von Streptavidin (1 mg/ml) in 0,1 mol/l Kaliumphosphatpuffer pH 7,0 getaucht, mit Puffer gespült und 1 h in einer Lösung von biotinylierter Glucoseoxidase in oben genanntem Puffer (1 mg/ml) inkubiert. Nach erneutem gründlichem Spülen mit Puffer und Wasser war der Sensor einsatzfähig.

Die Bestimmung des Analyten, in diesem Fall Glucose (10 mmol/l), erfolgte amperometrisch. Der Probelösung wurde Mediator, z.B. N-Nitrosoanilin in einer Konzentration von 1·10⁻³ mol/l, zugegeben und nach einer Inkubationszeit von 2 min. der Strom bei entsprechend dem Mediator angelegten Potential, hier 240 mV, gemessen.

Es wurden mit diesem Sensor nur sehr geringe Ströme bei Glucosezusatz erzielt, obwohl die GOD-Aktivität photometrisch nachweisbar war. Die in W0-A-92/10757 beschriebene Universalbindephase ist somit für elektrochemische Enzymsensoren nicht optimal. Die nächsten Beispiele beschreiben die Veränderung des Bindemoleküls, um zu einem für elektrochemische Anwendungen geeigneten Aufbau zu kommen.

### Beispiel 6

Die Goldträger entsprachen denen aus Beispiel 5. Die Träger wurden in 2·10⁻³ mol/l eines Gemisches aus Verbindung C (Mercaptonitrilotriessigsäure) und Verbindung A in Methanol 1 h lang inkubiert. Nach gründlichem, aufeinanderfolgendem Waschen in reinem Methanol und Wasser wurde 10 min. in einer 0,2%igen Lösung von NiCl₂ in Wasser inkubiert, wieder mehrmals in Wasser gewaschen und im Argonstrom getrocknet.

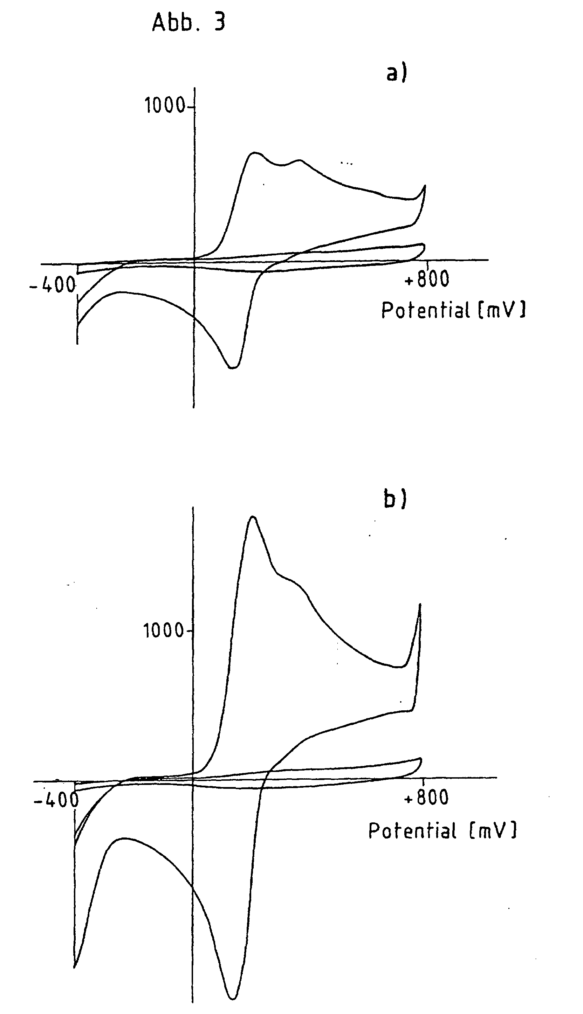

Die Schicht wurde mit Hilfe der Cyclovoltammetrie charakterisiert (vgl. Beispiel 5). Während beim Aufbringen der reinen Verbindung C der Umsatz redoxaktiver Substanzen wie K₄[(Fe(CN)₆] sehr stark erschwert war, führte die zusätzliche Bindung der Verbindung A auf der Goldoberfläche zu einer guten Reaktion des Mediators an der Elektrode (Abb. 3).

Auf Genebene modifizierte Glucoseoxidase, die C-terminal mit Histidinresten verlängert ist (vgl. Beispiel 1), wurde über Metallchelatbindung an die Festphase gebunden, indem man den modifizierten Träger 1 h in eine Lösung des Enzyms in 0,1 mol/l Kaliumphosphatpuffer pH 7,0 tauchte.

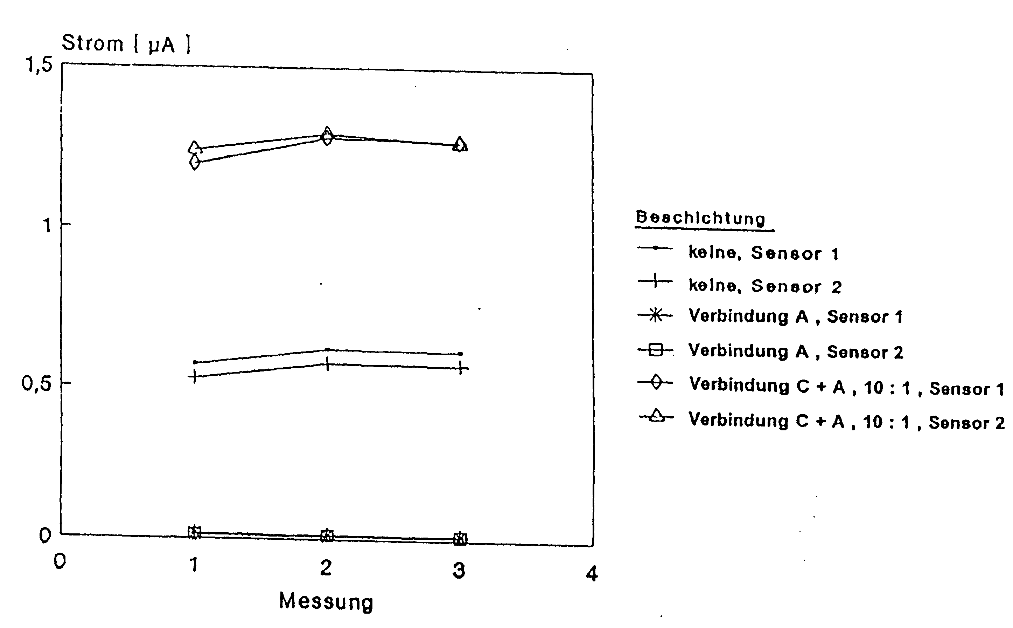

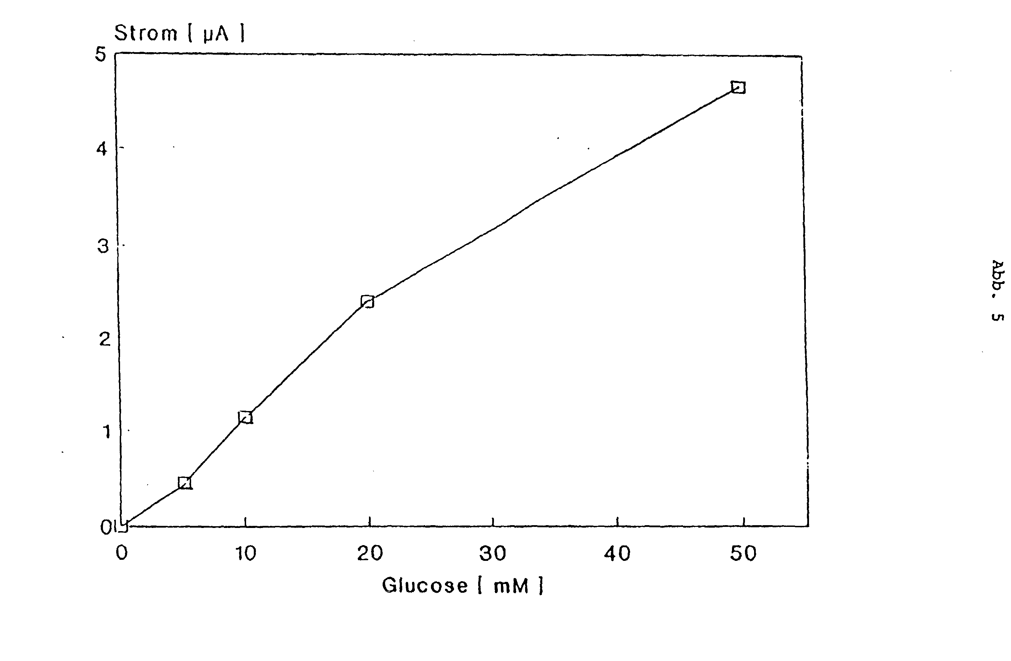

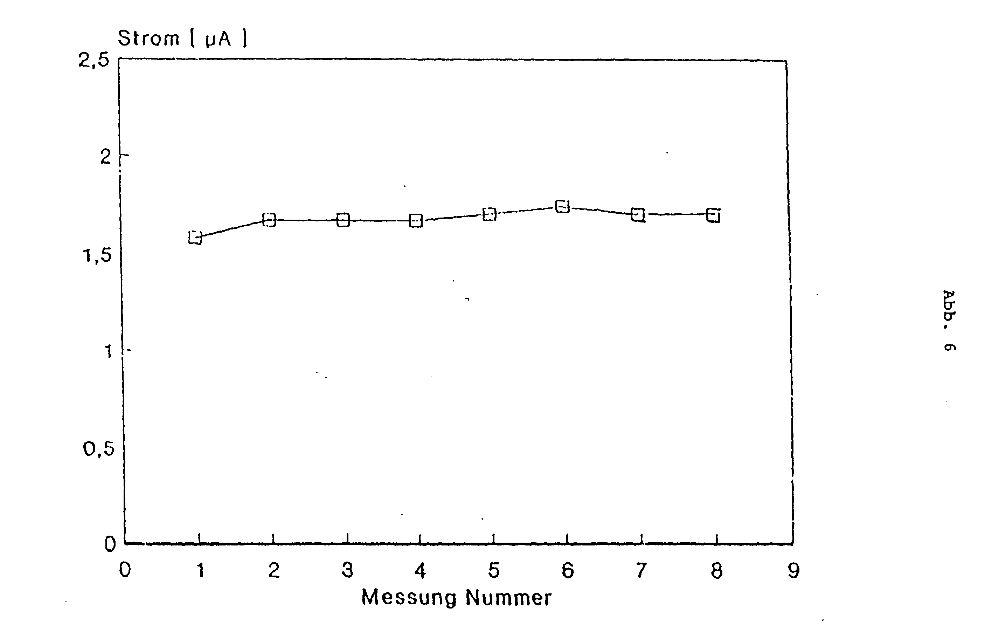

Die funktionsabhängige Bewertung fand wie in Beispiel 5 beschrieben mit einer 10 mmol/l Glucoselösung, N,N-Dimethyl-4-nitrosoanilin als Mediator und einem Potential von 240 mV statt. Abb. 4 zeigt., daß der gewählte Sensoraufbau zu einer deutlich nöheren Sensitivität führte als die direkte Immobilisierung des Enzyms auf der Goldoberfläche und die Immobilisierung auf der reinen Verbindung C. Ein molares Verhältnis der Verbindungen C und A von 10:1 erwies sich als am günstigsten. Im Gegensatz dazu kam es nicht zu einer Bindung des Enzyms an Oberflächen der reinen Verbindung A. Das Signal an einem Sensor, der mit einem Gemisch der Verbindungen A und C beschichtet war, verhielt sich proportional zum Gehalt der Glucose in der Probelösung (Abb. 5). Zudem war das Signal bei mehreren aufeinanderfolgenden Messungen stabil (Abb. 6).

### Beispiel 7

Die Goldträger entsprachen denen aus Beispiel 1. Sie wurden 1 h in einer Lösung der Verbindung D ("Maleimidlinker") und Verbindung A im molaren Verhältnis von 10:1 (beste Ergebnisse) und in einer Gesamtkonzentration von 1·10⁻³ mol/l in Methanol inkubiert. Nach gründlichem Waschen in Methanol und Wasser wurde im Argonstrom getrocknet.

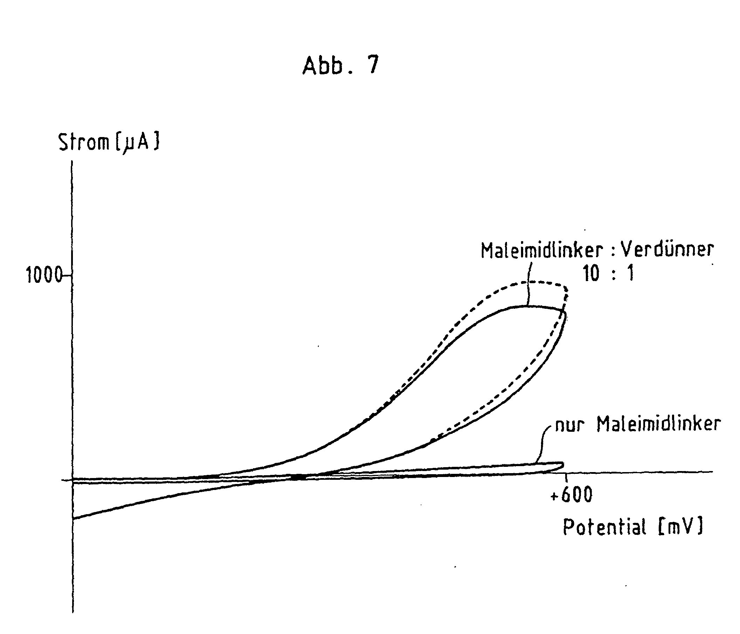

Das Cyclovoltammogramm mit 1 mmol/l K₄[Fe(CN)₆] zeigt, daß die dicht gepackte Schicht der reinen Verbindung D den Umsatz von N,N-Dimethylnitrosoanilin stark verringerte. Durch einen Zusatz der Verbindung A wurde diese Barriere aufgehoben, und die Oberfläche verhielt sich bezüglich der Redoxreaktion ähnlich aktiv wie reines Gold (Abb. 7).

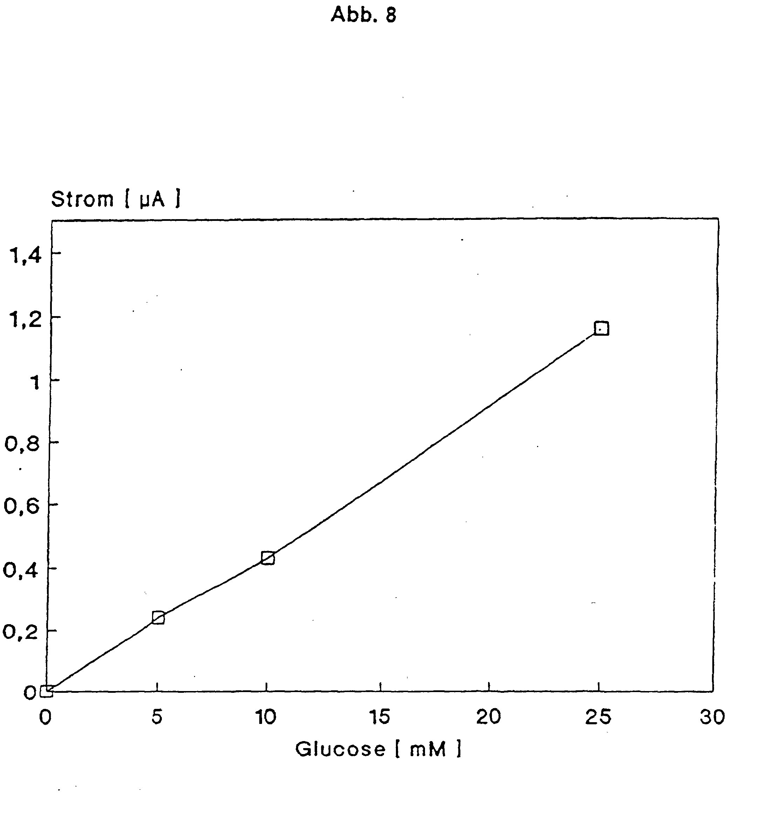

Anschließend erfolgte eine einstündige Inkubation des Trägers in einer Lösung von SH-modifizierter Glucoseoxidase GOD-Cys (1 mg/ml) in 0,1 mol/l Kaliumphosphatpuffer pH 7,0. Dabei wurde das Enzym kovalent über die Thiolgruppen an die Maleimid-Endgruppe des Linkers gebunden. Die Bestimmung des Analyten wurde, wie in Beispiel 5 beschrieben, durchgeführt. Abb. 8 zeigt die Zunahme des Stromes mit der Analytkonzentration.

### SEQ ID NO. 1

- ART DER SEQUENZ:: Nukleinsäure
- SEQUENZLÄNGE:: 18 Nukleotide

### SEQ ID NO. 2

- ART DER SEQUENZ:: Nukleinsäure
- SEQUENZLÄNGE:: 18 Nukleotide

### SEQ ID NO. 3

- ART DER SEQUENZ:: Nukleinsäure
- SEQUENZLÄNGE:: 27 Nukleotide

### SEQ ID NO. 4

- ART DER SEQUENZ:: Nukleinsäure
- SEQUENZLÄNGE:: 31 Nukleotide

### SEQ ID NO. 5

- ART DER SEQUENZ:: Nukleinsäure
- SEQUENZLÄNGE:: 18 Nukleotide

### SEQ ID NO. 6 :

- ART DER SEQUENZ:: Nukleinsäure
- SEQUENZLÄNGE:: 22 Nukleotide

(1) ALLGEMEINE ANGABEN:
   (i) ANMELDER:
      (A) NAME: Boehringer Mannheim GmbH
      (B) STRASSE: Sandhofer Strasse
      (C) ORT: Mannheim
      (E) LAND: DE
      (F) POSTLEITZAHL: 68298
      (G) TELEFON: 0621-7590
   (ii) BEZEICHNUNG DER ERFINDUNG: Elektrochemischer Sensor
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE P 44 30 023.9
      (B) ANMELDETAG: 24-AUG-1994
(2) ANGABEN ZU SEQ ID NO: 1:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (vi) URSPRÜNLICHE HERKUNFT:
      (A) ORGANISMUS: Aspergillus niger
   (vii) UNMITTELBARE HERKUNFT:
      (B) CLON(E): YEpL/AD-GOD
   (xi) SEQUENZBESCHREIBUNG: SEQ ID NO: 1:
   CGCGTCGGCC GGTCGACG 18
(2) ANGABEN ZU SEQ ID NO: 2:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (xi) SEQUENZBESCHREIBUNG: SEQ ID NO: 2:
   GATCCGTCGA CCGGCCGA 18
(2) ANGABEN ZU SEQ ID NO: 3:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (vi) URSPRÜNLICHE HERKUNFT:
      (A) ORGANISMUS: Aspergillus niger
   (vii) UNMITTELBARE HERKUNFT:
      (B) CLON(E): YEpL/GOD-(His)4Cys
   (xi) SEQUENZBESCHREIBUNG: SEQ ID NO: 3:
   CAGCACCACC ACCACTGTTG AATTCAG 27
(2) ANGABEN ZU SEQ ID NO: 4:
   (i) SEQUENZKENNZEICHEN:
      (A) LÄNGE: 31 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (xi) SEQUENZBESCHREIBUNG: SEQ ID NO: 4:
   CTGAATTCAA CAGTGGTGGT GGTGCTGCAT G 31
(2) ANGABEN ZU SEQ ID NO: 5:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (vi) URSPRÜNLICHE HERKUNFT:
      (A) ORGANISMUS: Aspergillus niger
   (vii) UNMITTELBARE HERKUNFT:
      (B) CLON(E): YEpL/GOD-Cys
   (xi) SEQUENZBESCHREIBUNG: SEQ ID NO: 5:
   CAGTGTTAAT GAATTCAG 18
(2) ANGABEN ZU SEQ ID NO: 6:
   (i) SEQUENZKENNZEICHEN:
      (A) LÄNGE: 22 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (xi) SEQUENZBESCHREIBUNG: SEQ ID NO: 6:
   CTGAATTCAT TAACACTGCA TG 22

## Patentansprüche

1. Elektrochemischer Sensor, enthaltend
ein Trägermaterial, das eine Edelmetalloberfläche aufweist,
eine adsorptiv an das Edelmetall gebundene und im wesentlichen lateral homogene Monoschicht auf der Oberfläche des Edelmetalls, wobei die Monoschicht Bindemoleküle umfaßt, die über Ankergruppen an das Edelmetall gebunden sind, wobei die Bindemoleküle über eine ionische Bindung, eine kovalente Bindung oder eine Metallchelatbindung mit einem enzymatisch aktiven Protein verknüpft sind, wobei der Belegungsgrad der Bindemoleküle auf dem Edelmetall geringer als der maximale Belegungsgrad ist und wobei das enzymatisch aktive Protein in einer dichtgepackten Schicht mit maximaler Belegungsdichte vorliegt.

2. Sensor nach Anspruch 1,
**dadurch gekennzeichnet,**
**daß** das Trägermaterial eine Gold- oder Palladiumoberfläche aufweist.

3. Sensor nach Anspruch 1 oder 2,
**dadurch gekennzeichnet,**
**daß** die Ankergruppen Thiol-, Disulfid-, oder/und Phosphingruppen sind.

4. Sensor nach einem der Ansprüche 1-3,
**dadurch gekennzeichnet,**
**daß** die Monoschicht weiterhin Verdünnungsmoleküle umfaßt, die über Ankergruppen adsorptiv an das Edelmetall gebunden und nicht mit einem enzymatisch aktiven Protein verknüpft sind.

5. Sensor nach Anspruch 4,
**dadurch gekennzeichnet,**
**daß** das molare Verhältnis von Bindemolekülen zu Verdünnungsmolekülen im Bereich von 1:100 bis 100:1 ist.

6. Sensor nach Anspruch 5,
**dadurch gekennzeichnet,**
**daß** das molare Verhältnis von Bindemolekülen zu Verdünnungsmolekülen im Bereich von 1:50 bis 50:1 ist.

7. Sensor nach Anspruch 5,
**dadurch gekennzeichnet,**
**daß** das molare Verhältnis von Bindemolekülen zu Verdünnungsmolekülen im Bereich von 1:20 bis 20:1 ist.

8. Sensor nach einem der vorherigen Ansprüche,
**dadurch gekennzeichnet,**
**daß** das enzymatisch aktive Protein aus der Gruppe der Enzyme ausgewählt ist, die eine Reaktion katalysieren, bei der Verbindungen entstehen oder/und verbraucht werden, die elektrochemisch detektierbar sind.

9. Sensor nach Anspruch 8,
**dadurch gekennzeichnet,**
**daß** das enzymatisch aktive Protein eine Oxidoreductase oder Hydrolase ist.

10. Sensor nach Anspruch 9,
**dadurch gekennzeichnet,**
**daß** das enzymatisch aktive Protein eine Glucose-Oxidase (E.C.1.1.3.4) ist.

11. Sensor nach einem der vorherigen Ansprüche,
**dadurch gekennzeichnet,**
**daß** das enzymatisch aktive Protein ein rekombinantes Enzym ist.

12. Sensor nach Anspruch 11,
**dadurch gekennzeichnet,**
**daß** das enzymatisch aktive Protein durch N- oder/und C-terminale Anfügung von einem oder mehreren Aminosäureresten, ausgewählt aus basischen Aminosäuren, sauren Aminosäuren, chelatbildenden Aminosäuren oder Cysteinresten modifiziert ist.

13. Sensor nach einem der vorherigen Ansprüche,
**dadurch gekennzeichnet,**
**daß** die Bindemoleküle über eine ionische Bindung mit dem enzymatisch aktiven Protein verknüpft sind, wobei die Bindung zwischen einer geladenen Endgruppe des Bindemoleküls und einem entgegengesetzt geladenen Bereich des enzymatisch aktiven Proteins erfolgt.

14. Sensor nach Anspruch 13,
**dadurch gekennzeichnet,**
**daß** die geladene Endgruppe des Bindemoleküls mindestens eine Aminofunktion umfaßt und der entgegengesetzt geladene Bereich des enzymatisch aktiven Proteins mindestens einen Aspartat- oder/und Glutamatrest umfaßt.

15. Sensor nach Anspruch 13,
**dadurch gekennzeichnet,**
**daß** die geladene Endgruppe des Bindemoleküls mindestens eine Carboxylat- oder/und Sulfonatfunktion umfaßt und der entgegengesetzt geladene Bereich des enzymatisch aktiven Proteins mindestens einen Arginin- oder/und Lysinrest umfaßt.

16. Sensor nach einem der Ansprüche 1-12,
**dadurch gekennzeichnet,**
**daß** die Bindemoleküle über eine kovalente Bindung mit dem enzymatisch aktiven Protein verknüpft sind, wobei die Bindung zwischen einer reaktiven Endgruppe des Bindemoleküls und einer reaktiven Gruppe des enzymatisch aktiven Proteins erfolgt.

17. Sensor nach Anspruch 16,
**dadurch gekennzeichnet, daß** die reaktive Endgruppe des Bindemoleküls eine Maleimidgruppe umfaßt und die reaktive Gruppe des enzymatisch aktiven Proteins einen Cysteinrest umfaßt.

18. Sensor nach einem der Ansprüche 1-12,
**dadurch gekennzeichnet,**
**daß** die Bindemoleküle über eine Metallchelatbindung mit dem enzymatisch aktiven Protein verknüpft sind, wobei die Bindung zwischen einer chelatbildenden Endgruppe des Bindemoleküls, einem Metallion und einem chelatbildenden Bereich des enzymatisch aktiven Proteins erfolgt.

19. Sensor nach Anspruch 18,
**dadurch gekennzeichnet,**
**daß** die chelatbildende Endgruppe des Bindemoleküls eine Di- oder Tricarboxylatgruppe umfaßt und der chelatbildende Bereich des enzymatisch aktiven Proteins mindestens einen Histidinrest umfaßt.

20. Sensor nach Anspruch 18 oder 19,
**dadurch gekennzeichnet,**
**daß** das Metallion ein Nickel-, Eisen-, Kobalt-, Kupfer- oder Zinkion ist.

21. Sensor nach einem der vorhergehenden Ansprüche,
**dadurch gekennzeichnet,**
**daß** das Bindemolekül zwischen der Ankergruppe und der zur Bindung mit dem enzymatisch aktiven Protein fähigen Endgruppe einen Spacer enthält.

22. Sensor nach Anspruch 21,
**dadurch gekennzeichnet,**
**daß** der Spacer eine gegebenenfalls Heteroatome enthaltende Alkylenkette mit einer Länge von 4-30 Atomen ist.

23. Verfahren zur Bestimmung eines Analyten in einer Probelösung,
**dadurch gekennzeichnet,**
**daß** man die Probelösung mit einem Sensor nach einem der Ansprüche 1-22 unter Bedingungen, die zu einer elektrochemisch nachweisbaren Reaktion des Analyten führen, kontaktiert und die Reaktion des Analyten durch eine elektrochemische Messung bestimmt.

24. Verfahren nach Anspruch 23,
**dadurch gekennzeichnet,**
**daß** man Glucose als Analyten bestimmt.

25. Verbindung der allgemeinen Formel
A-(Sp-X)ₙ (I),
worin A eine HS- oder H₂P-Gruppe und n 1 bedeutet oder A eine -SS-Gruppe und n 2 bedeutet,
Sp einen Spacer mit einer Kettenlänge von 4-30 Atomen bedeutet und X eine Maleimidgruppe oder eine Gruppe der Formel (II) 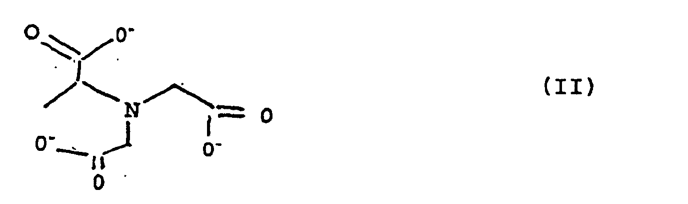 ist.

26. Verwendung einer Verbindung nach Anspruch 25 zur Herstellung eines elektrochemischen Sensors.

## Claims

1. Electrochemical sensor containing a supporting material which has a noble metal surface, an essentially laterally homogeneous monolayer on the surface of the noble metal which is adsorptively bound to the noble metal, wherein the monolayer comprises binding molecules which are bound to the noble metal via anchor groups,
wherein the binding molecules are linked to an enzymatically active protein by means of an ionic bond, a covalent bond or a metal-chelate bond and
wherein the degree of coverage of the binding molecules on the noble metal is less than the maximum degree of coverage and wherein the enzymatically active protein is present in a close-packed layer having a maximum coverage density.

2. Sensor as claimed in claim 1,
wherein
the supporting material has a gold or palladium surface.

3. Sensor as claimed in claim 1 or 2,
wherein
the anchor groups are thiol, disulphide or/and phospine groups.

4. Sensor as claimed in one of the claims 1-3,
wherein
the monolayer additionally contains diluent molecules which are adsorptively bound to the noble metal by means of anchor groups and are not linked to an enzymatically active protein.

5. Sensor as claimed in claim 4,
wherein
the molar ratio of binding molecules to diluent molecules is in the range of 1:100 to 100:1.

6. Sensor as claimed in claim 5,
wherein
the molar ratio of binding molecules to diluent molecules is in the range of 1:50 to 50:1.

7. Sensor as claimed in claim 5,
wherein
the molar ratio of binding molecules to diluent molecules is in the range of 1:20 to 20:1.

8. Sensor as claimed in one of the previous claims,
wherein
the enzymatically active protein is selected from the group of enzymes which catalyse a reaction in which compounds are formed or/and consumed which can be detected electrochemically.

9. Sensor as claimed in claim 8,
wherein
the enzymatically active protein is an oxidoreductase or hydrolase.

10. Sensor as claimed in claim 9,
wherein
the enzymatically active protein is a glucose oxidase (E.C.1.1.3.4).

11. Sensor as claimed in one of the previous claims,
wherein
the enzymatically active protein is a recombinant enzyme.

12. Sensor as claimed in claim 11,
wherein
the enzymatically active protein is modified by attaching at the N- or/and C-terminus one or several amino acid residues, selected from basic amino acids, acidic amino acids, chelate-forming amino acids or cysteine residues.

13. Sensor as claimed in one of the previous claims,
wherein
the binding molecules are linked to the enzymatically active protein by means of an ionic bond in which the bond is formed between a charged end group of the binding molecule and an oppositely charged region of the enzymatically active protein.

14. Sensor as claimed in claim 13,
wherein
the charged end group of the binding molecules comprises at least one amino function and the oppositely charged region of the enzymatically active protein comprises at least one aspartate or/and glutamate residue.

15. Sensor as claimed in claim 13,
wherein
the charged end group of the binding molecule comprises at least one carboxylate or/and sulfonate function and the oppositely charged region of the enzymatically active protein comprises at least one arginine or/and lysine residue.

16. Sensor as claimed in one of the claims 1-12,
wherein
the binding molecules are linked to the enzymatically active protein by means of a covalent bond in which the bond is formed between a reactive end group of the binding molecule and a reactive group of the enzymatically active protein.

17. Sensor as claimed in claim 16,
wherein
the reactive end group of the binding molecule comprises a maleimide group and the reactive group of the enzymatically active protein comprises a cysteine residue.

18. Sensor as claimed in one of the claims 1-12,
wherein
the binding molecules are linked to the enzymatically active protein by means of a metal chelate bond in which the bond is formed between a chelate-forming end group of the binding molecule, a metal ion and a chelate-forming region of the enzymatically active protein.

19. Sensor as claimed in claim 18,
wherein
the chelate-forming end group of the binding molecule comprises a dicarboxylate or tricarboxylate group and the chelate-forming region of the enzymatically active protein comprise at least one histidine residue.

20. Sensor as claimed in claim 18 or 19,
wherein
the metal ion is a nickel, iron, cobalt, copper or zinc ion.

21. Sensor as claimed in one of the previous claims,
wherein
the binding molecule contains a spacer between the anchor group and the end group capable of forming a bond with the enzymatically active protein.

22. Sensor as claimed in claim 21,
wherein
the spacer is an alkylene chain having a length of 4-30 atoms which may contain heteroatoms.

23. Method for the determination of an analyte in a sample solution,
wherein
the sample solution is contacted with a sensor as claimed in one of the claims 1-22 under conditions which lead to an electrochemically detectable reaction of the analyte and the reaction of the analyte is determined by an electrochemical measurement

24. Method as claimed in claim 23,
wherein
glucose is determined as the analyte.

25. Compound of the general formula
A-(Sp-X)n (I),
in which A denotes a HS or H₂P group and n denotes 1 or A denotes an -SS group and n denotes 2,
Sp denotes a spacer with a chain length of 4-30 atoms and X denotes a maleimide group or a group of formula (II)

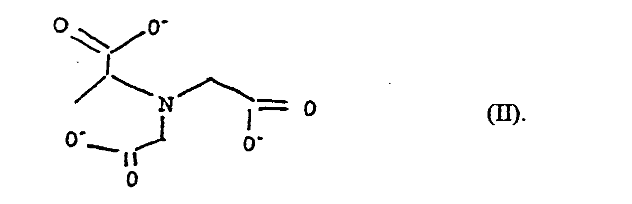

26. Use of a compound as claimed in claim 25 for manufacturing an electrochemical sensor.

## Revendications

1. Capteur électrochimique, contenant un matériau support, qui présente une surface en métal précieux, une monocouche sensiblement homogène latéralement et liée au métal précieux par adsorption sur la surface du métal précieux, la monocouche comprenant des molécules de liaison, qui sont liées au métal précieux par le biais de groupes d'ancrage, les molécules de liaison étant reliées par l'intermédiaire d'une liaison ionique, une liaison covalente ou une liaison de chélate métallique à une protéine enzymatiquement active, le degré d'occupation des molécules de liaison sur le métal précieux étant plus faible que le degré d'occupation maximal et la protéine enzymatiquement active se présentant sous la forme d'une couche condensée ou tassée avec une densité d'occupation maximale.

2. Capteur selon la revendication 1, **caractérisé en ce que** le matériau support présente une surface en or ou en palladium.

3. Capteur selon la revendication 1 ou 2, **caractérisé en ce que** les groupes d'ancrage sont des groupes thiol, disulfure et/ou phosphine.

4. Capteur selon l'une des revendications 1 à 3, **caractérisé en ce que** la monocouche comprend en outre des molécules de dilution, qui sont liées au métal précieux par adsorption par le biais de groupes d'ancrage et qui ne sont pas reliées à une protéine enzymatiquement active.

5. Capteur selon la revendication 4, **caractérisé en ce que** le rapport molaire des molécules de liaison aux molécules de dilution se situe dans la plage allant de 1 : 100 à 100 : 1.

6. Capteur selon la revendication 5, **caractérisé en ce que** le rapport molaire des molécules de liaison aux molécules de dilution se situe dans la plage allant de 1 : 50 à 50 : 1.

7. Capteur selon la revendication 5, **caractérisé en ce que** le rapport molaire des molécules de liaison aux molécules de dilution se situe dans la plage allant de 1 : 20 à 20 : 1.

8. Capteur selon l'une des revendications précédentes, **caractérisé en ce que** la protéine enzymatiquement active est choisie parmi le groupe des enzymes qui catalysent une réaction dans laquelle des composés détectables électrochimiquement apparaissent ou/et sont consommés.

9. Capteur selon la revendication 8, **caractérisé en ce que** la protéine enzymatiquement active est une oxydoréductase ou hydrolase.

10. Capteur selon la revendication 9, **caractérisé en ce que** la protéine enzymatiquement active est une glucose - oxydase (E.C.1.1.3.4.).

11. Capteur selon l'une des revendications précédentes, **caractérisé en ce que** la protéine enzymatiquement active est une enzyme recombinante.

12. Capteur selon la revendication 11, **caractérisé en ce que** la protéine enzymatiquement active est modifiée par adjonction terminale en N ou/et en C d'un ou de plusieurs résidus d'acides aminés, choisis parmi les acides aminés basiques, les acides aminés acides, les acides aminés chélateurs, ou les résidus de cystéine.

13. Capteur selon l'une des revendications précédentes, **caractérisé en ce que** les molécules de liaison sont reliées par une liaison ionique à la protéine enzymatiquement active, la liaison ayant lieu entre un groupe terminal chargé de la molécule de liaison et une zone de la protéine enzymatiquement active chargée de manière opposée.

14. Capteur selon la revendication 13, **caractérisé en ce que** le groupe terminal chargé de la molécule de liaison comprend au moins une fonction amine et **en ce que** la zone de la protéine enzymatiquement active chargée de manière opposée comprend au moins un résidu d'aspartate ou/et de glutamate.

15. Capteur selon la revendication 13, **caractérisé en ce que** le groupe terminal chargé de la molécule de liaison comprend au moins une fonction carboxylate ou/et sulfonate et **en ce que** la zone de la protéine enzymatiquement active chargée de manière opposée comprend au moins un résidu d'arginine ou/et de lysine.

16. Capteur selon l'une des revendications 1 à 12, **caractérisé en ce que** les molécules de liaison sont reliées par une liaison covalente à la protéine enzymatiquement active, la liaison se produisant entre un groupe terminal réactif de la molécule de liaison et un groupe réactif de la protéine enzymatiquement active.

17. Capteur selon la revendication 16, **caractérisé en ce que** le groupe terminal réactif de la molécule de liaison comprend un groupe maléimide et **en ce que** le groupe réactif de la protéine enzymatiquement active comprend un résidu de cystéine.

18. Capteur selon l'une des revendications 1 à 12, **caractérisé en ce que** les molécules de liaison sont reliées par une liaison de chélate métallique à la protéine enzymatiquement active, la liaison se produisant entre un groupe terminal chélateur de la molécule de liaison, un ion métallique et une zone chélatrice de la protéine enzymatiquement active.

19. Capteur selon la revendication 18, **caractérisé en ce que** le groupe terminal chélateur de la molécule de liaison comprend un groupe di- ou tricarboxylate et **en ce que** la zone chélatrice de la protéine enzymatiquement active comprend au moins un résidu d'histidine.

20. Capteur selon la revendication 18 ou 19, **caractérisé en ce que** l'ion métallique est un ion nickel, fer, cobalt, cuivre ou zinc.

21. Capteur selon l'une quelconque des revendications précédentes, **caractérisé en ce que** la molécule de liaison entre le groupe d'ancrage et le groupe terminal capable de liaison à la protéine enzymatiquement active comprend un écarteur ("spacer").

22. Capteur selon la revendication 11, **caractérisé en ce que** l'écarteur est une chaîne alkylène contenant éventuellement des hétéroatomes et ayant une longueur de 4 à 30 atomes.

23. Procédé de dosage d'un analyte dans une solution d'échantillon, **caractérisé en ce que** l'on met en contact la solution d'échantillon avec un capteur selon l'une des revendications 1 à 22 sous des conditions qui conduisent à une réaction électrochimiquement détectable de l'analyte et **en ce que** l'on détermine la réaction de l'analyte par une mesure électrochimique.

24. Procédé selon la revendication 23, **caractérisé en ce que** l'on dose du glucose à titre d'analyte.

25. Composé de formule générale :
A-(SP-X)ₙ (I),
dans laquelle A représente un groupe HS ou H₂P et n représente 1 ou A représente un groupe -SS- et n représente 2,
Sp représente un écarteur ayant une longueur de chaîne de 4 à 30 atomes et X est un groupe maléimide ou un groupe de formule (II)

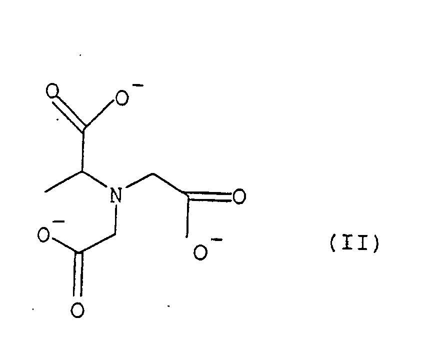

26. Utilisation d'un composé selon la revendication 25 pour la fabrication d'un capteur électrochimique.
